Europäisches Patentamt

**European Patent Office**

**Office européen des brevets**

(11) Publication number: **0 303 687 B1**

# EUROPEAN PATENT SPECIFICATION

(45) Date of publication of patent specification: **11.11.92**   (51) Int. Cl.[5]: **A61K 37/02**, A61K 39/395

(21) Application number: **88903027.6**

(22) Date of filing: **01.03.88**

(86) International application number:
**PCT/US88/00618**

(87) International publication number:
**WO 88/06452 (07.09.88 88/20)**

The file contains technical information submitted after the application was filed and not included in this specification

(54) **COMPOSITIONS FOR ENHANCING ADCC THERAPY.**

(30) Priority: **02.03.87 US 20275**
**23.06.87 US 65466**

(43) Date of publication of application:
**22.02.89 Bulletin 89/08**

(45) Publication of the grant of the patent:
**11.11.92 Bulletin 92/46**

(84) Designated Contracting States:
**CH DE FR GB IT LI SE**

(56) References cited:

JOURNAL OF IMMUNOLOGY, vol. 131, no. 6, December 1983 (US); A.F.LOPEZ et al., pp. 2983-2988&NUM;

PROCEEDINGS OF THE NATL. ACADEMY OF SCIENCE USA, vol. 81, June 1984; D.D.ADAMS et al., pp. 3506-3510&NUM;

(73) Proprietor: **GENETICS INSTITUTE, INC.**
**87 Cambridge Park Drive**
**Cambridge, Massachusetts 02140(US)**

(72) Inventor: **DE VILLIERS, Jean**
**49 Symphony Road Apartment 36**
**Boston, MA 02115(US)**
Inventor: **MUFSON, R., Allan**
**14 1/2 Fayette Street**
**Cambridge, MA 02139(US)**

(74) Representative: **Vossius & Partner**
**Siebertstrasse 4 P.O. Box 86 07 67**
**W-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 303 687 B1

SCIENCE, vol. 221, 24 August 1983, Washington, DC (US); Z.STEPLEWSKI et al., pp. 865-867&NUM;

JOURNAL OF CLINICAL INVESTIGATION, vol. 69, February 1982 (US); E.J.WING et al., pp. 270-276&NUM;

JOURNAL OF IMMUNOLOGY, vol. 130, no. 2, February 1983 (US); M.A.VADAS et al., pp. 795-799&NUM;

BLOOD, vol. 767, no. 1, January 1986 (US); D.METCALF et al., pp. 37-45&NUM;

# Description

The present invention relates to pharmaceutical compositions and the use thereof for selectively suppressing tumor cell growth in a human patient and specifically to the conjoint use of M-CSF and/or GM-CSF with certain monoclonal antibodies to destroy tumor cells.

The recognition and use of antibody dependent cell cytotoxicity (ADCC) reaction has been employed in developing cancer therapies, by using the antibodies alone or by using the antibodies to direct purported chemotherapeutic agents to the site of the cancer. See, e.g., published European Patent Application 194,851 which discloses treating a patient with beta-1,3-glucan lentinan followed by administration of anti-tumor monoclonal antibodies. PCT application no.WO87/00054 discloses attaching monoclonal antibodies to effector cells primed by gamma interferon and/or IL-2 and re-injecting a patient with the combination. For general information on the ADCC reaction, see also, A. F. Lopez et al, J. Immunol., 131(6):2983-2988 (1983); M. A. Vadas et al, J. Immunol., 130(2):795-799 (1983); T. J. Kipps et al, J. Exp. Med., 161:1-17 (1985). Lopez et al., supra, and Vadas et al., supra, describe e.g. the effects of CSFs on granulocytes in ADCC assays against TNP-coupled cells. Metcalf et al., Blood, 67(1):37-45(1986), describes ADCC assays using recombinant human GM-CSF.

Similar combination therapies were contemplated by Houghton et al., who speculated that "interferons, interleukin-2, tumor necrosis factor and other lymphokines could enhance the activity of effector cells directed to the tumor by MAbs." [Houghton, A.N., & D.A. Scheinberg, Sem. Oncol., 13: 165-179 (1984)]. Notwithstanding this generalized optimism, many of these factors to which Houghton et al. refer have in fact proven ineffective at augmenting immune-mediated tumor destruction.

Applicants have surprisingly found that M-CSF and GM-CSF are effective in potentiating the cytolysis of tumor cells by monocytes in an ADCC protocol. Specifically, M-CSF and GM-CSF are formulated into pharmaceutical compositions which prime macrophages such that they become capable of selectively lysing tumor cells in vivo. The compositions consist of two preparations which are locally separated. One such preparation is the colony stimulating factor ("the CSF") M-CSF and/or GM-CSF. The other preparation is a monoclonal antibody directed to an antigen associated with the tumor cells sought to be lysed. The present invention further contemplates the use of M-CSF and GM-CSF for priming macrophages either in vivo or ex vivo.

The preferred embodiment of this invention utilizes as the colony stimulating factor, M-CSF, also known as CSF-1, monocyte colony stimulating factor, and macrophage colony stimulating factor. Wing et al., J. Clin. Invest., 69:270-276(1982) describes the effect of mouse CSF on mouse macrophages against tumor cells. M-CSF induces colonies which contain primarily macrophages. It has been described in Wong et al., Science, 235:1504-1508 (1987) and publications by E. R. Stanley. Its production by recombinant DNA techniques is described in US-A-4,879,227. A truncated version is described in PCT/US86/00238.

Another CSF within the scope of this invention is GM-CSF, also known as granulocyte-macrophage colony stimulating factor. It is described in detail in Wong et al., Science, 228:810-815 (1985). Wong et al. also teach its production via recombinant DNA techniques. While GM-CSF had been recognized as exhibiting significant in vitro activity on the various hematopoietic progenitor cells, its use in treatment of clinical conditions remains prospective. See also, EPO Pub. No. 0,183,350 and PCT Application WO87/02060.

M-CSF displays surprising efficacy over other lymphokines affecting monocytes and macrophages in ADCC experiments in priming those cells to lyse target tumor cells. The term "priming" or "primed" macrophage in this context refers to the effect on a responsive macrophage of exposure to a lymphokine. The macrophage becomes primed, and is therefore capable of becoming fully cytolytic (or activated) upon exposure to a monoclonal antibody. [See, e.g., D. Adams, Ann. Rev. Immunol. (1984).]

It has been found that M-CSF can prime macrophages to kill tumor cells in a significantly shorter time period than other immune regulators, and the effector cells require less monoclonal antibody to achieve such kill. Similarly, GM-CSF also shows efficacious results in such a protocol.

A preparation consisting of a monoclonal antibody directed to antigens associated with the tumor cells to be lysed (e.g., a cell surface antigen) is another essential component of this invention. This monoclonal antibody may be selected from murine antibody isotypes IgG3, IgG2A, IgG2B, and human antibody isotype IgG1.

One aspect of the present invention provides a pharmaceutical composition for selectively killing tumor cells in a human patient by priming macrophages pre-incubated with M-CSF and/or GM-CSF and co-administering the primed cells with the above-identified monoclonal antibodies to tumor-associated antigens to the patient ; i.e. the macrophage priming occurs ex vivo.

Another aspect of the invention provides a pharmaceutical composition for selectively killing tumor cells in a human patient without necessitating extracorporeal treatment. This is accomplished by directly co-administering to the patient an effective dose of M-CSF and/or GM-CSF and an effective dose of a monoclonal antibody selected from among the above isotypes ; i.e. the macrophage priming occurs in vivo.

The M-CSF and GM-CSF employed in the present invention are desirably purified human proteins, free from association with other contaminating proteins. The M-CSF and GM-CSF so employed include the natural proteins, recombinant versions thereof, and derivatives and analogs thereof, which may contain amino acid deletions, substitutions and/or insertions, but which retain the characteristic biological activity and are encoded by cDNAs capable of hybridizing to cDNAs for the naturally occurring versions. Also included are naturally-occurring isotypes or allelic variations in the protein or its coding sequence resulting from expression of the protein in different members of a species.

Several specific monoclonal antibodies of the human isotype IgG1 and the murine antibody isotypes IgG2 and IgG3 are described in EPA 194,851. The type of tumor cells which are destroyed by this method depends on the particular antigen to which the monoclonal antibodies are directed. For example, co-administration of M-CSF and/or GM-CSF with a murine IgG3 antibody developed against human lung carcinoma cells directs the primed macrophages to localize around, and thus lyse, those lung tumor cells. Similar antibodies of the murine isotypes IgG2A and IgG2B or human isotype IgG1 directed to other tumor types, such as breast, colon and liver, are available, or can be developed by one of skill in the art. Examples of existing monoclonal antibodies which are potentially useful according to the method include: PM4E9917, a murine IgG2a directed against a liver carcinoma cell surface antigen [R. Carlson et al., J. Clin. Invest., 76:40-46 (1985)]; 791T/36, a murine IgG2b directed against a colon carcinoma cell surface antigen [L.G. Durrant, Cancer Res., 46:3543-3549 (1986)]; and 083-17-1A, a murine IgG2a directed against a colon carcinoma cell surface antigen [H. Koprowski et al, Somat. Cell Genet., 5:957-972 (1979)].

As used herein, the term "co-administration" encompasses serial or sequential administration of the components of the method, including pre-administration of one or more components in favor of the others. For example, the monoclonal antibody is administered to the patient as a first step and subsequently M-CSF and/or GM-CSF is administered, or the CSF is first administered, and subsequently a monoclonal antibody and the other CSF are administered, or the CSF is administered prior to the administration of the monoclonal antibody or the monoclonal antibody and the CSF are administered simultaneously.

Thus, when antibody and M-CSF and/or GM-CSF are administered separately to the patient, the CSF causes in vivo priming of the macrophages in the body and the antibody becomes associated with the tumor cells. The primed macrophages then specifically recognize tumor cell/antibody complex.

Alternatively, M-CSF and GM-CSF may be used to prime monocytes in vitro, which are then readministered to the patient who has been pretreated with antibody and the same specific cytolysis may occur. In this regimen, antibody may also be given after re-introduction of the in vitro priming of monocytes by CSF.

In yet another embodiment, M-CSF and/or GM-CSF are used to prime the monocytes in vitro by incubation with the CSF and then subsequently "armed" in vitro by attachment to the antibody. Attachment of the primed macrophages to the monoclonal antibody ("arming") in vitro may be accomplished in a number of conventional ways. [See, e.g., Z. Steplewski et al, Science, 221:865-867 (1983)]. The complex is then reintroduced into a patient to obtain the same specific tumor cell cytolysis. Other orders of administration are also encompassed by this term.

The use of these compositions may find particular application as a therapy to control metastatic spread of tumor cells following surgical removal of a primary tumor. As well as the components enumerated specifically herein, this method may utilize other substances that are active on tumor-associated monoclonal antibodies.

The components of this invention can be systemically administered parenterally. Alternatively, these components may be administered intravenously. Where desired for treatment, it may also be possible to administer one or more components of the method subcutaneously. When systemically administered, the therapeutic preparations of the monoclonal antibody and CSF and optional second CSF for use in this invention are in the form of a pyrogen-free, parenterally acceptable aqueous solutions. The preparation of such a parenterally acceptable protein solution, having due regard to pH, isotonicity and stability, is within the skill of the art.

The dosage regimen involved in the use of these compositions for suppressing susceptible tumors will be determined by the attending physician considering various factors which modify the action of drugs, e.g., the condition, body weight, sex, and diet of the patient, the severity of any infection, time of administration and other clinical factors. The CSF component should be administered in a dosage insufficient to cause a systemic toxic reaction, but sufficient to elicit the potentiating response on the effector cells.

Generally, the daily regimen should be in the range of 200-1000 micrograms of CSF polypeptide, or 50 to 5000 units (ie, a unit being the concentration of polypeptide which elicits a half maximal response in an in vitro colony forming assay or a macrophage activation assay). The dosage cited above may be adjusted to compensate for whether the method involves the use of only one CSF or a combination of CSFs. The effective dosages of the monoclonal antibody are in the range of 0.1 to 1 mg/kg body weight daily, depending on the physician's estimate of tumor size and the extent of metastasis. Progress of the treated patient can be monitored by periodic assessment of the metastasis.

The following example illustrates the significant advantages in the employment of M-CSF in ADCC reactions according to the present invention.

EXAMPLE I

Peripheral blood monocytes are prepared by adherence to tissue culture plates by conventional methods. Approximately $5 \times 10^5$ to $5 \times 10^6$ adhered monocytes/well are incubated at 37°C for either 24, 48 or 72 hours in 20 ng recombinant M-CSF per tissue culture medium,as verified by amino acid analysis. Control plates of monocytes were incubated under the same conditions and times but without the addition of M-CSF.

The monocytes from each plate are added to an ADCC protocol, performed as described in Herlyn et al, Cellular Immunology, 92:105-114(1985), employing monoclonal antibodies of the murine isotype IgG3 prepared against colon carcinoma surface antigens and colon carcinoma cell line, LS-174 [NeoRx Inc.]. The colon carcinoma cells employed as the target cells in the ADCC protocol are prelabelled with [3]H thymidine and all cell death is measured by release of [3]H thymidine. The ADCC reaction was allowed to run for 5 days.

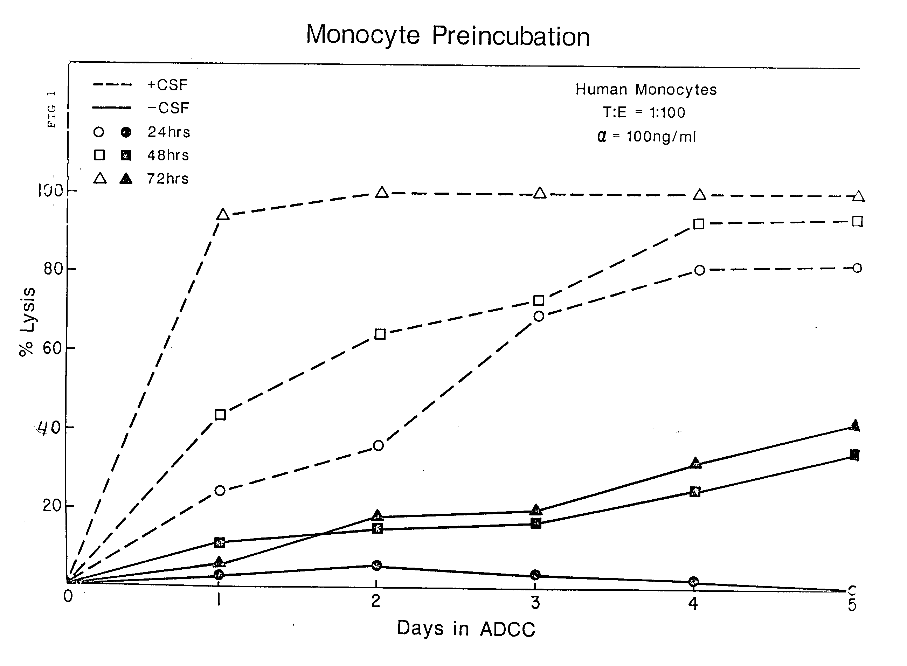

Fig. 1 shows the results of the ADCC reaction by plotting days in ADCC vs. percentage of target cell lysis. The target to effector cell ratio in the experiment of Fig 1 is 1:100; however, the same results can be obtained with a T:E ratio of 1:10. The antibody concentration in the experiment of Fig. 1 is 100 ng/ml. The adhered monocytes incubated in M-CSF clearly caused more target cell death at all pre-incubation times than did the controls. In general, M-CSF pretreated monocytes kill approximately two and one-half times as many target cells as untreated monocytes. The adhered monocytes which were preincubated in M-CSF for 72 hours allowed the fastest rate of killing to occur in the ADCC reaction, achieving maximum killing within 24 hours.

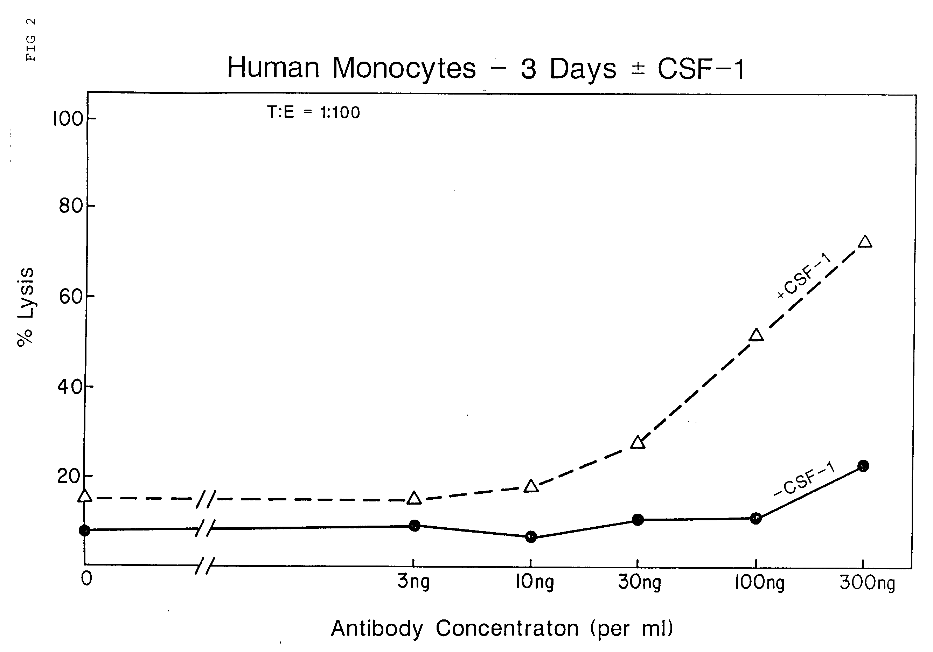

Fig. 2 demonstrates that monocytes pretreated with M-CSF for 72 hours required 10-fold less antibody to acheive target cell killing than did untreated monocytes in the ADCC protocol employing a T:E ratio of 1:100. Similar results were demonstrated with T:E: ratios as low as 1:10.

**Claims**

1. A pharmaceutical composition for selectively lysing tumor cells in a human patient characterized in that the composition consists of two preparations which are locally separated and comprising:
   (a) a monoclonal antibody directed to an antigen associated with said tumor cells and selected from murine antibody isotypes IgG3, IgG2A and IgG2B and human antibody isotype IgG1; and
   (b) a colony stimulating factor selected from M-CSF and GM-CSF.

2. The composition of claim 1 in which the colony stimulating factor is M-CSF.

3. The composition of claim 1 in which the colony stimulating factor is GM-CSF.

4. The composition according to claim 1 wherein the composition contains both M-CSF and GM-CSF.

5. Use of (a) a monoclonal antibody directed to an antigen associated with tumor cells and selected from murine antibody isotypes IgG3, IgG2A and IgG2B, and human antibody isotype IgG1; and
   (b) a colony stimulating factor selected from M-CSF and GM-CSF
   for the preparation of a pharmaceutical product comprising a combination of the monoclonal antibody and the colony stimulating factor suitable for separate sequential use of the monoclonal antibody and the colony stimulating factor in the treatment of cancer.

6. The use of claim 5 wherein the colony stimulating factor is M-CSF.

7. The use of claim 5 wherein the colony stimulating factor is GM-CSF.

8. The use of claim 5 wherein the colony stimulating factor is M-CSF and GM-CSF.

**Patentansprüche**

1. Arzneimittel zur selektiven Lyse von Tumorzellen in einem Menschen, dadurch gekennzeichnet, daß das Arzneimittel aus zwei räumlich getrennten Präparationen besteht, umfassend:

(a) einen gegen ein mit den Tumorzellen verbundenes Antigen gerichteter monoclonaler Antikörper, ausgewählt aus Maus-Antikörper-Isotypen IgG3, IgG2A und IgG2B und menschlichem Antikörper-Isotyp IgG1; und

(b) einen Kolonie-stimulierenden Faktor, ausgewählt aus M-CSF und GM-CSF.

2. Arzneimittel nach Anspruch 1, in dem der Kolonie-stimulierende Faktor M-CSF ist.

3. Arzneimittel nach Anspruch 1, in dem der Kolonie-stimulierende Faktor GM-CSF ist.

4. Arzneimittel nach Anspruch 1, wobei das Arzneimittel sowohl M-CSF als auch GM-CSF enthält.

5. Verwendung

(a) eines gegen ein mit Tumorzellen verbundenes Antigen gerichteten monoclonalen Antikörpers, ausgewählt aus Maus-Antikörper-Isotypen IgG3, IgG2A und IgG2B und menschlichem Antikörper-Isotyp IgG1; und

(b) eines Kolonie-stimulierenden Faktors, ausgewählt aus M-CSF und GM-CSF

zur Herstellung eines Arzneimittels, umfassend eine Kombination des monoclonalen Antikörpers und des Koloniestimulierenden Faktors, die für eine getrennte, sequentielle Verwendung des monoclonalen Antikörpers und des Kolonie-stimulierenden Faktors bei der Behandlung von Krebs geeignet ist.

6. Verwendung nach Anspruch 5, wobei der Kolonie-stimulierende Faktor M-CSF ist.

7. Verwendung nach Anspruch 5, wobei der Kolonie-stimulierende Faktor GM-CSF ist.

8. Verwendung nach Anspruch 5, wobei der Kolonie-stimulierende Faktor M-CSF und GM-CSF ist.

**Revendications**

1. Composition pharmaceutique pour la lyse sélective de cellules tumorales chez un patient humain, caractérisée en ce que la composition est constituée par deux préparations qui sont séparées localement, et comprenant :

(a) un anticorps monoclonal dirigé contre un antigène associé auxdites cellules tumorales et choisi parmi les isotypes d'anticorps murins IgG3, IgG2A et IgG2B et l'isotype d'anticorps humain IgG1; et

(b) un facteur de stimulation de colonie choisi parmi M-CSF et GM-CSF.

2. La composition de la revendication 1 dans laquelle le facteur de stimulation de colonie est M-CSF.

3. La composition de la revendication 1 dans laquelle le facteur de stimulation de colonie est GM-CSF.

4. La composition selon la revendication 1 dans laquelle la composition contient à la fois M-CSF et GM-CSF.

5. L'utilisation de

(a) un anticorps monoclonal dirigé contre un antigène associé à des cellules tumorales et choisi parmi les isotypes d'anticorps murins IgG3, IgG2A et IgG2B et l'isotype d'anticorps humain IgG1; et

(b) un facteur de stimulation de colonie choisi parmi M-CSF et GM-CSF,

pour la fabrication d'un produit pharmaceutique comprenant une combinaison de l'anticorps monoclonal et du facteur de stimulation de colonie, adapté à l'utilisation séquentielle séparée de l'anticorps monoclonal et du facteur de stimulation de colonie pour le traitement du cancer.

6. L'utilisation selon la revendication 5 dans laquelle le facteur de stimulation de colonie est M-CSF.

7. L'utilisation selon la revendication 5 dans laquelle le facteur de stimulation de colonie est GM-CSF.

8. L'utilisation selon la revendication 5 dans laquelle le facteur de stimulation de colonie est M-CSF et GM-CSF.

Monocyte Preincubation

FIG 1

EP 0 303 687 B1

FIG 2

Human Monocytes – 3 Days ± CSF–1

T:E = 1:100

+CSF–1

–CSF–1

% Lysis

Antibody Concentraton (per ml)